# EUROPÄISCHE PATENTSCHRIFT

(11) **EP 2 637 025 B1**
(45) Veröffentlichungstag und Bekanntmachung des Hinweises auf die Patenterteilung: **13.04.2016**
(21) Anmeldenummer: 13156498.1
(22) Anmeldetag: 25.02.2013
(51) Int. Cl.: G01N 33/86

(54) **Screeningverfahren zum Auffinden von Proben mit einer Störung der Funktionalität der GPIb-Von Willebrand Faktor-Interaktion**
Screening method for detecting samples with impaired GPIb-Von Willebrand factor interaction functionality
Procédé de criblage destiné à la recherche d'échantillons ayant une défaillance de fonctionnalité de l'interaction du facteur GPIb de Willbrand

(30) Priorität: 05.03.2012 EP 12158022
(43) Veröffentlichungstag der Anmeldung: 11.09.2013
(73) Patentinhaber: Siemens Healthcare Diagnostics Products GmbH, 35041 Marburg (DE)
(72) Erfinder: Patzke, Juergen, Dr., 35043 Marburg (DE)

(56) Entgegenhaltungen:
- EP-A1- 1 850 133
- WO-A1-2009/026551
- WO-A2-01/02853
- WO-A2-2009/007051
- VANHOORELBEKE KAREN ET AL: "A reliable and reproducible ELISA method to measure ristocetin cofactor activity of von Willebrand factor", THROMBOSIS AND HAEMOSTASIS, SCHATTAUER GMBH, DE; US, Bd. 83, Nr. 1, 1. Januar 2000 (2000-01-01) , Seiten 107-113, XP000974940, ISSN: 0340-6245

## Beschreibung

Die vorliegende Erfindung liegt auf dem Gebiet der Gerinnungsdiagnostik und betrifft ein Screening-Verfahren zur Bestimmung einer gestörten von Willebrand Faktor (VWF)-und/oder GPIb-Interaktion in einer Probe.

Der von Willebrand-Faktor (VWF) ist ein hochmolekulares, multimeres Glykoprotein im Blutplasma, das wichtige Funktionen in dem Prozess der primären Hämostase hat. Der VWF verfügt unter anderem über Bindungsstellen für Kollagen und für das Glykoprotein Ib (GPIb), das auf der Thrombozytenoberfläche lokalisiert ist. GPIb ist ein integrales Membranprotein, das mit einem weiteren integralen Membranprotein, dem Glykoprotein IX (GPIX), den Glykoprotein Ib-IX-Rezeptorkomplex in der Thrombozytenmembran bildet. GPIb ist ein zweikettiges Molekül, das aus einer schweren Kette mit einer apparenten molekularen Masse von etwa 145 kDa (synonym: heavy chain, alpha-Kette oder GPIbα) und einer leichten Kette mit einer apparenten molekularen Masse von etwa 22 kDa (synonym: light chain, beta-Kette oder GPIbβ) besteht, welche über Disulfidbrücken miteinander verbunden sind (Lopez, J.A. et al., Cloning of the α chain of human platelet glycoprotein Ib: A transmembrane protein with homology to leucine-rich α2-glycoprotein. Proc. Natl. Acad. Sci USA 1987, 84: 5615-5619). Glycocalicin ist ein Fragment der GPIbα-Kette, das proteolytisch von dem intakten Rezeptor in der Thrombozytenmembran abgespalten wird. Glycocalicin ist im Plasma nachweisbar. Erhöhte Konzentrationen von freiem Glycocalicin im Plasma weisen auf eine Störung der Plättchenfunktion hin (Beer, J.H. et al., Glycocalicin: A New Assay - The Normal Plasma Levels And Its Potential Usefulness in Selected Diseases. Blood 1994, 83(3): 691-702).

Im Falle einer Gefäßverletzung werden Kollagenoberflächen exponiert, an die VWF bindet. Infolge der Bindung an Kollagen und unter dem Einfluss der erhöhten Scherkräfte, die auf den Kollagen-gebundenen VWF einwirken, wird der VWF so verändert oder aktiviert, dass er an das aminoterminale Ende der schweren Kette des GPIb (GPIbα) im GPIb-IX-Rezeptorkomplex der Thrombozytenmembran binden kann. Auf diese Weise fängt der aktivierte VWF vorbeiströmende Thrombozyten ein, so dass sich am Ort der Verletzung ein erstes Agglomerat aus VWF, Kollagen und Thrombozyten bildet. In der Folge werden die Thrombozyten aktiviert und damit auch die plasmatische Gerinnung in Gang gesetzt, die letztlich, nach mehreren Verstärkungskaskaden und der Anlagerung weiterer Thrombozyten, zum Wundverschluss führt. Störungen der VWF-GPIb-Interaktion führen zu einer erhöhten Blutungsneigung.

Qualitative oder quantitative Störungen des VWF sind die Ursache eines sogenannten von Willebrand-Syndroms (synonym: von Willebrand Disease, VWD), eines der häufigsten erblichen Blutungsleiden. Zur Diagnose eines von Willebrand-Syndroms stehen verschiedene Screeningverfahren zur Verfügung, wie z.B. die Bestimmung der Blutungszeit (BT), quantitative Verfahren zur Bestimmung der VWF-Antigenkonzentration (VWF:Ag), wie z.B. ELISA-Verfahren, sowie Verfahren zur Bestimmung der VWF-Aktivität, wie z.B. die Ristocetininduzierte Plättchenagglutination (VWF:RCo).

Bei der neusten Generation von funktionellen Testen zur Bestimmung der VWF-Aktivität wird die GPIbα-Bindefähigkeit des VWF bestimmt.

Es sind Teste bekannt, bei denen wildtypisches GPIbα verwendet wird und die Bindung des VWF an GPIbα in Gegenwart von Ristocetin bestimmt wird (WO 01/02853 A2; Vanhoorelbeke, K. et al., A reliable von Willebrand factor: Ristocetin cofactor enzyme-linked immunosorbent assay to differentiate between type 1 and type 2 von Willebrand disease. Semin Thromb Hemost. 2002, 28(2): 161-165; Federici, A.B. et al., A sensitive ristocetin co-factor activity assay with recombinant glycoprotein Ibα for the diagnosis of patients with low von Willebrand factor levels. Haematologica 2004, 89(1): 77-85).

Es sind andere Teste bekannt, bei denen sog. gain-of-function Mutationen von GPIbα verwendet werden, die bekanntermaßen eine höhere Affinität für VWF aufweisen und stärker mit VWF interagieren als wildtypisches GPIbα-Protein. Bei diesen Testen kann die Bindung des VWF an das mutierte GPIbα in Abwesenheit von Ristocetin bestimmt werden (WO 2009/007051 A2 oder WO 2009/026551 A1).

Defekte des GPIb-Proteins verursachen ebenfalls Blutungsleiden. Gain-of-function Mutationen des GPIb-Proteins sind die Ursache für das Platelet-Type-von-Willebrand-Syndrom (PT-VWD), ein autosomal dominant vererbtes Blutungsleiden. Die Substitution des Methionin-Restes an Position 239 der GPIbα-Kette durch einen Valin-Rest (M239V) wurde von Russell & Roth beschrieben (Russell, S.D. & Roth, G.J.,Pseudo-von Willebrand Disease: A mutation in the platelet glycoprotein Ibα gene associated with a hyperactive surface receptor. Blood 1993, 81(7): 1787-1791). Auch Mutationen an Position 233 der GPIbα-Kette können ein PT-VWD verursachen (Matsubara, Y. et al., Identification of a novel point mutation in platelet glycoprotein Ibα, Gly to Ser at residue 233, in a Japanese family with platelet-type von Willebrand disease. Journal of Thrombosis and Haemostasis 2003, 1: 2198-2205).

Wird bei einem Patienten eine erhöhte Blutungsneigung diagnostiziert, ist es erforderlich, die Ursache der Störung zu erkennen, um eine geeignete Therapie einleiten zu können. Aufgrund der vielen möglichen Störungen, die eine erhöhte Blutungsneigung hervorrufen können, ist es in der klinischen Diagnostik wünschenswert über Screening-Teste zu verfügen, die es zunächst ermöglichen die Funktionalität bestimmter Teilbereiche des Gerinnungssystems zu untersuchen. Kann mit Hilfe eines solchen Screening-Tests eine Störung in einem bestimmten Teilbereich lokalisiert werden, können gezielte Einzelteste durchgeführt werden, um die genaue Ursache zu spezifizieren. Wird mit Hilfe eines Screening-Tests keine Störung in einem bestimmten Teilbereich festgestellt, so kann auf die Durchführung spezifischer Einzelteste verzichtet werden.

Der vorliegenden Erfindung lag daher die Aufgabe zugrunde, ein Screening-Verfahren bereit zu stellen, das es ermöglicht, Störungen der VWF-GPIb-Interaktion festzustellen. Ein solches Verfahren sollte gleichermaßen sensitiv für Störungen des VWF sowie für Störungen des GPIb-Proteins sein.

Störungen der VWF-GPIb-Interaktion können zum Beispiel hervorgerufen werden durch
a) quantitative oder qualitative Störungen des VWF-Proteins, wie z.B. abnormale Mangelzustände, Fehlen der großen Multimere, fehlende Faktor VIII-Bindungsfähigkeit;
b) VWF-Inhibitoren, wie z.B. Autoantikörper gegen den VWF, die die Bindung des VWF an GPIb verhindern oder erhöhte Konzentrationen von Glycocalicin im Plasma, welches die VWF-Bindungsstellen belegt und somit die VWF Aktivität mindert oder VWF-inhibierende Therapeutika wie z.B. ARC1779, ein VWF-bindendes Aptamer, oder AJW200, ein humanisierter monoklonaler anti-VWF Antikörper (Firbas, C. et al., Targeting von Willebrand factor and platelet glycoprotein Ib receptor. Expert Rev. Cardiovasc. Ther. 2010, 8(12): 1689-1701) oder GPIb-Fragment, welches als Therapeutikum eingesetzt wird (Hennan, J.K. et al., Pharmacologic inhibition of platelet vWF-GPIbα interaction prevents coronary artery thrombosis. Thromb Haemost 2006, 95: 469-75);
c) VWF-Aktivatoren;
d) qualitative Störungen des GPIb-Proteins, wie z.B. gain-of-function Mutationen, die eine höhere Affinität für VWF aufweisen und daher den Abbau von VWF beschleunigen;
e) GPIb-Inhibitoren, wie z.B. Autoantikörper gegen GPIb, die die Bindung des VWF an GPIb verhindern oder Therapeutika wie z.B. H6B4-Fab, das Fab-Fragment eines humanisierten monoklonalen anti-GPIbα-Antikörpers (siehe ebenfalls Firbas, C. et al.);
f) GPIb-Aktivatoren (z.B. Thrombin).

Die Aufgabe wird dadurch gelöst, dass die Probe eines Patienten mit isoliertem GPIbα-Protein, mit VWF-Protein und mit einer Festphase, die mit einem GPIbα-spezifischen Antikörper assoziiert ist, in Kontakt gebracht wird, und die Komplexbildung zwischen VWF-Protein, GPIbα-Protein und der Festphase bestimmt wird. Ist die Komplexbildung gegenüber dem Normal vermindert oder erhöht, liegt eine Störung der VWF-GPIb-Interaktion vor. Solche Proben sollten dann mit Hilfe von spezifischen Einzeltesten gezielt auf VWF- bzw. GPIb-Störungen untersucht werden.

Gegenstand der vorliegenden Erfindung ist daher ein Verfahren zum Auffinden einer Probe mit einer gestörten VWF-GPIb-Interaktion, wobei
a. die Probe mit GPIbα-Protein, mit VWF-Protein und mit einer Festphase, die mit einem Antikörper mit Spezifität für das isolierte GPIbα-Protein assoziiert ist, in Kontakt gebracht wird, und
b. die Komplexbildung zwischen VWF-Protein, GPIbα-Protein und der Festphase bestimmt wird.

Das Verfahren erkennt Störungen der VWF-GPIb-Interaktion sowohl auf der VWF- wie auch auf der GPIb-Seite.

Der Begriff "Probe" umfasst biologische Flüssigkeiten insbesondere von Menschen und Tieren, wie Blut, Plasma oder Serum.

Bei dem in dem erfindungsgemäßen Verfahren verwendeten GPIbα-Protein kann es sich um ein rekombinant oder synthetisch hergestelltes GPIbα-Protein handeln. Zur Herstellung von rekombinantem GPIbα-Protein eignen sich bekannte prokaryontische oder eukaryontische Expressionssysteme, wie z.B. die Expression in Bakterien (z.B. E. coli), in Hefen (z.B. Saccharomyces cerevisiae, Pichia pastoris), in pflanzlichen, tierischen oder humanen Zellkulturen. Zur Herstellung von synthetischem GPIbα-Protein eignen sich bekannte Techniken zur in vitro Proteinsynthese, wie z. B. Festphasensynthesen (z.B. Merrifield-Synthese). Bevorzugterweise handelt es sich bei dem in dem erfindungsgemäßen Verfahren verwendeten GPIbα-Protein um rekombinant hergestelltes GPIbα-Protein, das in einer Kultur humaner Zellen, bevorzugt in einer Kultur humaner embryonaler Nierenzellen (HEK-Zellen), hergestellt wurde.

Bevorzugterweise wird GPIbα-Protein dem Testansatz in einer solchen Menge zugesetzt, dass eine Endkonzentration von weniger als 1,4 µg/mL GPIbα im Testansatz, besonders bevorzugt von weniger als 0,7 µg/mL GPIbα im Testansatz erhalten wird.

Das in dem erfindungsgemäßen Verfahren verwendete GPIbα-Protein kann am N-Terminus mit der homologen humanen GPIbα-Signalsequenz MPLLLLLLLLPSPLHP (SEQ ID NO: 2, auch als Aminosäurereste -16 bis -1 bezeichnet) fusioniert sein. Alternativ kann das verwendete GPIbα-Protein am N-Terminus mit einer heterologen Signalsequenz fusioniert sein, d.h. mit einem Polypeptid, das üblicherweise nicht in dem humanen GPIbα-Polypeptid vorhanden ist, die aber in dem gewählten Expressionssystem die Expression und/oder Sekretion des rekombinant exprimierten GPIbα-Proteins positiv beeinflusst. Eine geeignete heterologe Signalsequenz ist z. B. MPLQLLLLLILLGPGNSLQLWDTWADEAEKALGPLLARDRR (SEQ ID NO: 3).

Weiterhin kann das in dem erfindungsgemäßen Verfahren verwendete GPIbα-Protein am C-Terminus mit einem oder mehreren Affinitäts-Tags fusioniert sein, die die Bindung des z.B. rekombinant exprimierten Proteins an einen Affinitätsträger ermöglichen, wodurch z.B. die Reinigung von rekombinant exprimiertem GPIbα-Protein ermöglicht wird. Bevorzugt sind kleine Affinitäts-Tags mit einer Länge von nicht mehr als 12 Aminosäuren. Besonders bevorzugt sind Affinitäts-Tags aus der Gruppe His-Tag, Flag-Tag, Arg-Tag, c-Myc-Tag und Strep-Tag. Geeignete Affinitätsträger, die mit hoher Affinität an ein Affinitäts-Tag binden, sind z.B. spezifische Antikörper, immobilisierte Kationen (z. B. Ni²⁺ mit Affinität für His-Tags) oder andere Typen von Bindungspartnern (z.B. Streptavidin mit Affinität für Strep-Tags).

In einer Ausführungsform des erfindungsgemäßen Verfahrens ist das isolierte GPIbα-Protein humanes wildtypisches GPIbα-Protein (SEQ ID NO: 1) oder ein funktionelles Fragment davon. Wird humanes wildtypisches GPIbα-Protein oder ein funktionelles Fragment davon verwendet, wird dem Testansatz weiterhin Ristocetin, Botrocetin oder eine Ristocetin-äquivalente Substanz hinzugefügt, damit in vitro eine Bindung von gelöstem VWF an das wildtypische GPIbα-Protein oder Fragmente davon induziert wird.

In einer anderen Ausführungsform ist das isolierte GPIbα-Protein mutiert und enthält -verglichen mit der Wildtypsequenz des humanen GPIbα-Proteins (SEQ ID NO: 1)-mindestens die Aminosäurereste 1-268 und an mindestens zwei der Positionen 233, 235 und 239 jeweils eine Substitution Xaa. Bevorzugterweise bestehen die Substitutionen Xaa des Glycin-Rests an Position 233 und des Methionin-Rest an Position 239 der GPIbα-Kette aus einem Valin-Rest (G233V bzw. M239V) oder einem Serin-Rest (G233S bzw. M239S). Jede beliebige Kombination der unterschiedlichen Substitutionen Xaa an den beiden Positionen ist möglich. Besonders bevorzugt ist die Kombination G233V/M239V. Die Substitution Xaa des Asparaginsäurerestes an Position 235 besteht vorzugsweise aus einem Tyrosin-Rest (D235Y). Bei den genannten Mutationen handelt es sich um gain-of-function Mutationen, die eine signifikant höhere Affinität für VWF aufweisen und stärker mit VWF interagieren als wildtypisches GPIbα-Protein. Wird eine solche Mutation verwendet, wird dem Testansatz weder Ristocetin, Botrocetin noch eine Ristocetin-äquivalente Substanz hinzugefügt.

Bei dem in dem erfindungsgemäßen Verfahren verwendeten VWF-Protein kann es sich um isolierten hochmolekularen, multimerem VWF handeln. Humanes VWF-Monomer wird in vivo zunächst als 2813 Aminosäuren langes Vorläuferprotein synthetisiert. Durch intrazelluläre Prozessierung entstehen VWF-Multimere, die über 20 000 kDa groß werden können. Diese Multimere bestehen aus linear angeordneten, über Disulfidbrücken untereinander verbundenen 275 kDa großen, 2050 Aminosäuren langen VWF-Monomeren. Im Plasma zirkuliert der VWF in globulärer Form von unterschiedlich großen Multimeren von etwa 500 kDa (Dimer) bis über 15 000 kDa Größe. Das in dem erfindungsgemäßen Verfahren verwendete isolierte VWF-Protein kann entweder aus Spenderplasmen gewonnen werden, oder es kann mit Hilfe von dem Fachmann bekannten Verfahren rekombinant exprimiert werden. Alternativ kann das VWF-Protein auch in natürlicher Form, z.B. in Form eines Normalplasmas dem Testansatz zugegeben werden.

Bevorzugterweise wird VWF-Protein dem Testansatz in einer solchen Menge zugesetzt, dass eine Endkonzentration von 0,1-20% der Norm VWF im Testansatz, besonders bevorzugt von 0,5-10% der Norm im Testansatz erhalten wird.

Bei dem in dem erfindungsgemäßen Verfahren verwendeten Antikörper mit Spezifität für das isolierte GPIbα-Protein kann es sich um jeden Antikörper handeln, der das verwendete GPIbα-Protein in dem Testansatz spezifisch erkennt. Grundsätzlich geeignet sind GPIbα-Antikörper, die an ein Epitop des GPIbα-Proteins binden. Sofern ein rekombinantes und mit einem Affinitäts-Tag fusioniertes GPIbα-Protein verwendet wird, ist auch ein Affinitäts-Tag-spezifischer Antikörper, der an das Affinitätstag spezifisch bindet, geeignet. Der Begriff Antikörper umfasst auch Antikörper-Fragmente, die über dieselbe Antigen-Spezifität verfügen wie der vollständige Antikörper.

Der Antikörper ist mit einer Festphase assoziiert. Der Begriff "assoziiert" ist breit zu verstehen und umfasst beispielsweise eine kovalente und eine nicht-kovalente Bindung, eine direkte und eine indirekte Bindung, die Adsorption an eine Oberfläche und den Einschluss in eine Vertiefung. Bei einer kovalenten Bindung ist der Antikörper über eine chemische Bindung an die Festphase gebunden. Ein Beispiel für eine nicht-kovalente Bindung ist die Oberflächenadsorption. Neben einer direkten Bindung an die Festphase kann der Antikörper auch indirekt über spezifische Wechselwirkung mit anderen spezifischen Bindungspartnern an die Festphase gebunden sein, z.B. über spezifische Wechselwirkung mit einem weiteren Antikörper.

Der Begriff "Festphase" im Sinne dieser Erfindung beinhaltet einen Gegenstand, der aus porösem und/oder nicht porösem, wasserunlöslichem Material besteht und die unterschiedlichsten Formen aufweisen kann, wie z.B. Gefäß, Röhrchen, Mikrotitrationsplatte (ELISA-Platte), Kugel, Mikropartikel, Stäbchen, Streifen, Filter- oder Chromatographiepapier, etc. In der Regel ist die Oberfläche der Festphase hydrophil oder kann hydrophil gemacht werden. Die Festphase kann aus den unterschiedlichsten Materialien bestehen wie z.B. aus anorganischen und/oder aus organischen Materialien, aus synthetischen, aus natürlich vorkommenden und/oder aus modifizierten natürlich vorkommenden Materialien. Beispiele für Festphasenmaterialien sind Polymere wie z.B. Cellulose, Nitrocellulose, Zelluloseacetat, Polyvinylchlorid, Polyacrylamid, vernetzte Dextranmoleküle, Agarose, Polystyrol, Polyethylen, Polypropylen, Polymethacrylat oder Nylon; Latex; Keramik; Glas; Metalle, insbesondere Edelmetalle wie Gold und Silber; Magnetit; Mischungen oder Kombinationen derselben.

Die Festphase kann einen Überzug aus einer oder mehreren Schichten aufweisen, z.B. aus Proteinen, Kohlehydraten, lipophilen Substanzen, Biopolymeren, organischen Polymeren oder Mischungen hiervon, um beispielsweise die unspezifische Bindung von Probenbestandteilen an die Festphase zu unterdrücken oder zu verhindern, oder um beispielsweise Verbesserungen zu erreichen hinsichtlich der Suspensionsstabilität von partikulären Festphasen, der Lagerstabilität, der formgebenden Stabilität oder der Resistenz gegen UV-Licht, Mikroben oder sonstige zerstörend wirkender Agenzien.

Durch das Inkontaktbringen von isoliertem GPIbα-Protein mit VWF-Protein und mit einer Festphase, die mit einem Antikörper mit Spezifität für das isolierte GPIbα-Protein assoziiert ist, bildet sich ein Komplex aus den drei Komponenten. Sind in der Patientenprobe Substanzen enthalten, die diese Komplexbildung beeinflussen, z.B. GPIb- oder VWF-Inhibitoren oder Aktivatoren oder Glycocalicin oder enthält die Probe funktionell gestörte GPIb- oder VWF-Proteine, die mit den funktionsfähigen zugegebenen Proteinen bei der Komplexbildung konkurrieren, wird eine gegenüber dem Normal veränderte Komplexbildung gemessen.

In einer bevorzugten Ausführungsform des erfindungsgemäßen Verfahrens ist der Antikörper mit Spezifität für das isolierte GPIbα-Protein mit einer partikulären Festphase, bevorzugt mit Latexpartikeln assoziiert. Die Komplexbildung zwischen VWF-Protein, GPIbα-Protein und der Festphase kann dann durch Messung der Agglutination der partikulären Festphase bestimmt werden. Zur quantitativen Bestimmung der Agglutinationsreaktion, die mit der Komplexbildung und damit mit der in der Probe vorhandenen GPIb- und VWF-Aktivität korreliert, kann z.B. die Lichtstreuung an den Partikelaggregaten über die Messung der Streulichintensität (Nephelometrie) oder über die Messung der Trübung des Mediums (Turbidimetrie) genutzt werden.

In einer anderen Ausführungsform des erfindungsgemäßen Verfahrens ist der Antikörper mit Spezifität für das isolierte GPIbα-Protein mit einer nicht-partikulären Festphase assoziierten, bevorzugterweise mit der Oberfläche einer Mikrotiterplatte. Die Komplexbildung zwischen VWF-Protein, GPIbα-Protein und der Festphase kann dann durch Messung der VWF-Menge, die über den Komplex an die Festphase gebunden wird bestimmt werden. Zur Bestimmung der VWF-Menge, die über den Komplex an die Festphase gebunden wurde, kann beispielweise ein anti-VWF-Antikörper verwendet werden, der direkt oder indirekt mit einer Komponente eines signalbildenden Systems assoziiert ist, und damit die Quantifizierung der gebundenen VWF-Menge erlaubt.

Ein weiterer Gegenstand der vorliegenden Erfindung ist ein Testkit zur Durchführung eines erfindungemäßen Verfahrens enthaltend ein erstes Reagenz, das isoliertes GPIbα-Protein enthält, ein zweites Reagenz, das VWF-Protein enthält und ein drittes Reagenz, das eine Festphase, bevorzugt eine partikuläre Festphase, die mit einem Antikörper mit Spezifität für das isolierte GPIbα-Protein assoziiert ist, enthält.

In einer Ausführungsform enthält das erste Reagenz humanes wildtypisches GPIbα-Protein oder ein funktionelles Fragment davon. In einer anderen Ausführungsform enthält das erste Reagenz ein mutiertes GPIbα-Protein, das verglichen mit der Wildtypsequenz des humanen GPIbα-Proteins (SEQ ID NO: 1) mindestens die Aminosäurereste 1-268 enthält und an mindestens zwei der Positionen 233, 235 und 239 jeweils eine Substitution Xaa aufweist.

Ferner kann das Testkit ein viertes Reagenz, das Ristocetin oder Botrocetin enthält, enthalten.

Ferner kann das Testkit ein fünftes Reagenz, das einen anti-VWF-Antikörper enthält, enthalten.

Die Reagenzien können in flüssiger oder lyophilisierter Form bereitgestellt werden. Für den Fall, dass ein Reagenz als Lyophilisat vorliegt, kann das Testkit zusätzlich ein zur Suspendierung des Lyophilisats erforderliches Lösemittel enthalten, wie z.B. destilliertes Wasser oder einen geeigneten Puffer.

In einer weiteren Ausführungsform kann zur besseren Standardisierung der Ergebnisse eine Normalisierung durchgeführt werden. Dazu wird das Ergebnis der Probe durch das Ergebnis eines Normalplasmas dividiert. Somit äußert sich eine besonders starke VWF-GPIb-Interaktion durch ein Ratio größer 1 und eine verminderte VWF-GPIb-Interaktion durch ein Ratio kleiner 1. Weiterhin kann auch eine Kalibration mit einem Kalibrator durchgeführt werden. Dieser Kalibrator enthält von Willebrand Faktor. Die Ergebnisse müssen jedoch in künstlichen Einheiten ausgedrückt werden, da in der Probe die GPIb-VWF Interaktion erfasst wird. Ein Inhibitor gegen GPIb erniedrigt die Ergebnisse des Tests, obwohl die VWF-Aktivität normal bleibt.

Wird mit Hilfe des erfindungsgemäßen Verfahrens eine Probe mit gestörter VWF-GPIb-Interaktion identifiziert, sind Folgeteste zur näheren Eingrenzung der Störung durchzuführen. Hierbei kann eine Variante des erfindungsgemäßen Verfahrens durchgeführt werden, bei der entweder das GPIbα oder der VWF oder beides dem Testansatz in starkem Überschuss zugegeben werden. Wird GPIbα in starkem Überschuss zugegeben, reagiert der Test nur noch auf Störungen der VWF-Aktivität. Wird VWF in starkem Überschuss zugegeben, reagiert der Test nur noch auf Störungen der GPIb-Seite der Interaktion. Werden GPIbα und VWF in starkem Überschuss zugegeben, könnten zum Beispiel Autoantikörper gegen den festphasengebundenen Fänger-Antikörper detektiert werden, die die Bindung des GPIbα verhindern.

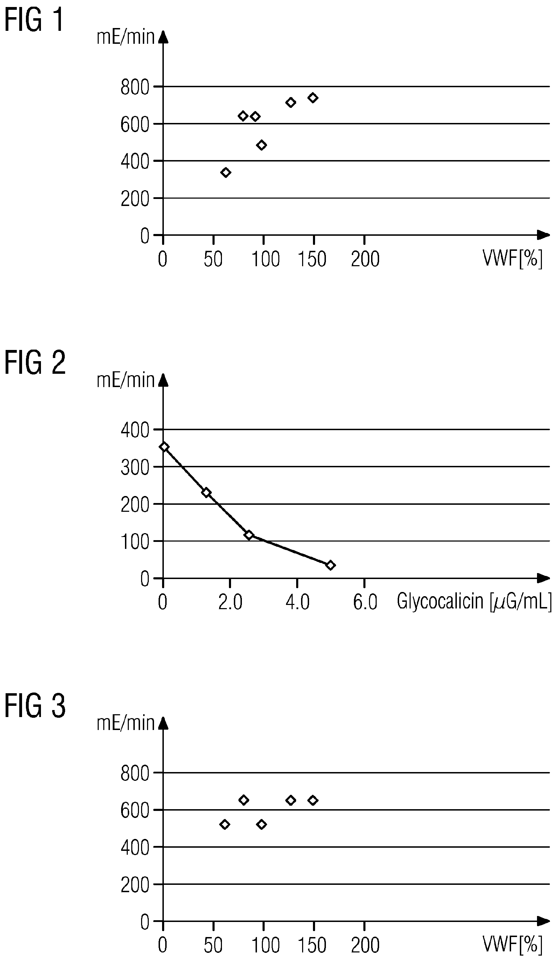

Eine mögliche Störung auf der GPIb-Seite ist eine Erhöhung oder Erniedrigung der Glycocalicin-Konzentration in der Probe. In Gegenwart eines starken Überschusses von VWF und wenn andere Störungen ausgeschlossen werden können, kann das Verfahren auch zur Bestimmung des Glycocalicin-Gehaltes der Probe benutzt werden (Figur 2), wobei eine Kalibration mit einem Glycocalicin Standard notwendig ist. Ein starker Überschuss an VWF kann zum Beispiel durch die Zugabe von 20 µL eines Reagenzes mit 1000 oder 2000% VWF-Aktivität erreicht werden, so dass eine VWF-Aktivität von 99% bzw. 197% der Norm im Testansatz vorliegt. Ein starker Überschuss an GPIbα kann zum Beispiel durch die Zugabe von 13 µL eines Reagenzes mit 35 µg/mL GPIbα-Protein erreicht werden, so dass eine Konzentration von 2,2 µg/mL GPIbα-Protein im Testansatz vorliegt.

Ein weiterer Gegenstand der vorliegenden Erfindung ist daher ein Verfahren zur Bestimmung der Glycocalicin-Konzentration in einer Probe, wobei die Probe mit isoliertem GPIbα-Protein, mit VWF-Protein und mit einer Festphase, die mit einem Antikörper mit Spezifität für das isolierte GPIbα-Protein assoziiert ist, in Kontakt gebracht wird, und die Komplexbildung zwischen VWF-Protein, GPIbα-Protein und der Festphase bestimmt wird. Zur Bestimmung der Glycocalicin-Konzentration ist es erforderlich, dass dem Testansatz eine Menge VWF-Protein zugegeben wird, so dass eine VWF-Aktivität von mindestens 50 %, bevorzugterweise von mehr als 100 % der Norm im Testansatz erhalten wird. Bevorzugterweise wird eine Kalibration mit Kalibratoren durchgeführt, die unterschiedliche Glycocalicin-Konzetrationen enthalten.

### Figurenbeschreibung

### Figur 1

Figur 1 zeigt, dass ein Latexpartikelagglutinationstest basierend auf dem erfindungsgemäßen Verfahren sensitiv ist für verschiedene VWF-Aktivitäten.

### Figur 2

Figur 2 zeigt, dass ein Latexpartikelagglutinationstest basierend auf dem erfindungsgemäßen Verfahren sensitiv ist für unterschiedliche Glycocalicin-Konzentrationen.

### Figur 3

Figur 3 zeigt, dass ein Latexpartikelagglutinationstest basierend auf dem erfindungsgemäßen Verfahren nicht sensitiv ist für verschiedene VWF-Aktivitäten, wenn dem Testansatz ein VWF-Überschuss hinzugefügt ist.

### BEISPIELE

- **Beispiel 1**: **Erfindungsgemäßes Screeningverfahren zum Auffinden von Proben mit einer gestörten VWF-GPIb-Interaktion**

Als Probe wurde humanes Citratplasma von 6 verschiedenen Spendern verwendet. 60 µL Probe wurden mit 20 µL eines VWFenthaltenden Plasmas (91% der Norm VWF, Kontroll Plasma N, Siemens Healthcare Diagnostics Products GmbH, Marburg, Deutschland), 13 µL einer Lösung enthaltend ein rekombinantes gain-of-function GPIbα-Proteinfragment (Aminosäuren 1-285 mit Aminosäureaustauschen G233V und M239V; 8,4 µg/mL) und 70 µL eines NaCl-Puffers vermischt und 2 Minuten inkubiert. Anschließend wurden dem Testansatz 40 µL eines Latexpartikel-Reagenzes zugegeben, welches Latexpartikel enthält, die mit Antikörpern gegen GPIbα beschichtet sind.

Die Extinktion des Reaktionsgemisches wurde bei 570 nm turbidimetrisch erfasst. Durch die Bindung von VWF an das GPIbα und die Bindung des GPIbα-Proteins an die Latexpartikel kommt es zur Partikelagglutination. Diese Agglutination führt zu einer Extinktionszunahme, deren maximale Geschwindigkeit ermittelt wird (mE/min).

Wie in Figur 1 dargestellt, ist das Verfahren sensitiv für unterschiedliche VWF-Aktivitäten. Die Agglutinationsgeschwindigkeit ist um so größer, je höher die VWF-Aktivität in einer Plasmaprobe ist.

In einem anderen Versuch wurden anstelle von Spenderplasmen verschiedene Verdünnungen einer Glycocalicin-Lösung als Probe verwendet (0, 1,2, 2,5 und 5 µg/mL Glycocalicin). Die Glycocalicin-Lösung wurde durch eine Plasmin-Behandlung gewaschener Thrombozyten hergestellt.

Wie in Figur 2 dargestellt, ist das Verfahren sensitiv für unterschiedliche Glycocalicin-Konzentrationen. Die Agglutinationsgeschwindigkeit ist um so größer, je geringer die Glycocalicin-Konzentration in einer Probe ist.

In einem anderen Versuch wurden anstelle von Spenderplasmen ein normaler Plasmapool (Kontrollplasma N) verwendet, der mit verschiedenen Mengen eines anti-GPIb-Antikörpers, der die Bindung von GPIbα an VWF hemmt, angereichert wurde (Antikörper AK2; siehe Hayata, K. et al., A new binding assay of von Willebrand factor and glycprotein Ib using solid-phase biotinylated platelets. J Pharmacol Sci 2008, 108: 217-221). Weiterhin wurde das Citratplasma eines Spenders mit leicht erniedrigter VWF-Aktivität gemessen. Die Ergebnisse sind in Tabelle 1 zusammengefasst. Das Verfahren ist demnach sensitiv für Faktoren, die die VWF-GPIb-Bindung inhibieren. Die Agglutinationsgeschwindigkeit ist um so geringer, je mehr Inhibitor in einer Probe enthalten ist.

**Tabelle 1**

| **Probe** | **Agglutinationsrate (mE/min)** |
|---|---|
| Normaler Plasmapool | 633 |
| Normaler Plasmapool + 10 µg/mL AK2 Antikörper | 266 |
| Normaler Plasmapool + 20 µg/mL AK2 Antikörper | 133 |
| Plasma mit leicht erniedrigter VWF-Aktivität (63.1 %) | 348 |

- **Beispiel 2:**: **Als Folgetest geeignete Abwandlung des erfindungsgemäßen Screeningverfahren**

Wie in Beispiel 1 wurde als Probe humanes Citratplasma von 5 verschiedenen Spendern verwendet. Anstelle des VWFenthaltenden Normalplasmas (91% der Norm VWF) wurden dem Testansatz jedoch 20 µL eines Plasmas zugegeben, das durch Zugabe eines VWF-Konzentrats (Hämate, CSL Behring GmbH, Marburg, Deutschland) eine VWF-Aktivität von 2000% der Norm aufwies.

Wie in Figur 3 dargestellt, ist das Verfahren durch die Gegenwart eines starken Überschusses an VWF nicht mehr sensitiv für verschiedene VWF-Aktivitäten in den Spenderproben. Ein derartiges Verfahren ist nur noch sensitiv für Störungen der VWF-GPIb-Interaktion, die auf der GPIb-Seite liegen.

## Patentansprüche

1. Verfahren zum Auffinden einer Patientenprobe mit einer gestörten VWF-GPIb-Interaktion, wobei
a. eine Blut-, Plasma- oder Serumprobe eines Patienten mit isoliertem GPIbα-Protein, mit VWF-Protein und mit einer Festphase, die mit einem Antikörper mit Spezifität für das isolierte GPIbα-Protein assoziiert ist, in Kontakt gebracht wird, und
b. die Komplexbildung zwischen VWF-Protein, GPIbα-Protein und der Festphase bestimmt wird.

2. Verfahren nach Anspruch 1, wobei das GPIbα-Protein humanes wildtypisches GPIbα-Protein oder ein funktionelles Fragment davon ist und wobei die Probe zusätzlich mit Ristocetin oder Botrocetin in Kontakt gebracht wird.

3. Verfahren nach Anspruch 1, wobei das GPIbα-Protein mutiert ist und verglichen mit der Wildtypsequenz des humanen GPIbα-Proteins mindestens die Aminosäurereste 1-268 enthält und an mindestens zwei der Positionen 233, 235 und 239 jeweils eine Substitution Xaa aufweist (SEQ ID NO: 1) und wobei die Probe nicht mit Ristocetin oder Botrocetin in Kontakt gebracht wird.

4. Verfahren nach Anspruch 3, wobei das mutierte GPIbα-Protein eine Substitution aus der Gruppe G233V, G233S, D235Y, M239V und M239S aufweist.

5. Verfahren nach einem der vorherigen Ansprüche, wobei die Probe mit einer Menge von GPIbα-Protein in Kontakt gebracht wird, so dass im Testansatz, in dem die Komplexbildung bestimmt wird, eine Endkonzentration von weniger als 1,4 µg/mL GPIbα-Protein, besonders bevorzugt von weniger als 0,7 µg/mL GPIbα-Protein erhalten wird.

6. Verfahren nach einem der vorherigen Ansprüche, wobei die Probe mit einer Menge von VWF-Protein in Kontakt gebracht wird, so dass im Testansatz, in dem die Komplexbildung bestimmt wird, eine Endkonzentration von 0,1 - 20% der Norm VWF-Aktivität, besonders bevorzugt von 0,5 - 10% der Norm VWF-Aktivität erhalten wird.

7. Verfahren nach einem der vorherigen Ansprüche, wobei die Festphase eine partikuläre Festphase ist.

8. Verfahren nach Anspruch 6, wobei die Komplexbildung zwischen VWF-Protein, GPIbα-Protein und der Festphase anhand der Agglutination der partikulären Festphase bestimmt wird.

9. Verfahren nach einem der Ansprüche 1 bis 6, wobei die Festphase eine nicht-partikuläre Festphase ist, bevorzugt die Oberfläche einer Mikrotitrationsplatte.

10. Verfahrens nach Anspruch 9, wobei die Komplexbildung zwischen VWF-Protein, GPIbα-Protein und der Festphase anhand der Bestimmung von Festphasen-gebundenem VWF-Protein bestimmt wird.

## Claims

1. Process for finding a patient sample having a disordered VWF-GPIb interaction, comprising
a. contacting a blood, plasma or serum sample of a patient with isolated GPIbα protein, with VWF protein and with a solid phase associated with an antibody specific for said isolated GPIbα protein, and
b. determining formation of a complex between VWF protein, GPIbα protein and the solid phase.

2. Process according to Claim 1, wherein the GPIbα protein is human wild-type GPIbα protein or a functional fragment thereof, and wherein the sample is in addition contacted with ristocetin or botrocetin.

3. Process according to Claim 1, wherein the GPIbα protein has been mutated and, compared to the wild-type sequence of human GPIbα protein, contains at least the amino acid residues 1-268 and has an Xaa substitution in each of at least two of positions 233, 235 and 239 (SEQ ID NO: 1), and wherein the sample is contacted with neither ristocetin nor botrocetin.

4. Process according to Claim 3, wherein the mutated GPIbα protein has a substitution from the group consisting of G233V, G233S, D235Y, M239V and M239S.

5. Process according to any of the preceding claims, wherein the sample is contacted with an amount of GPIbα protein such that a final concentration of less than 1.4 µg/ml GPIbα protein, particularly preferably of less than 0.7 µg/ml GPIbα protein, is obtained in the assay mix in which complex formation is determined.

6. Process according to any of the preceding claims, wherein the sample is contacted with an amount of VWF protein such that a final concentration of 0.1 - 20% of the norm VWF activity, particularly preferably of 0.5 - 10% of the norm VWF activity, is obtained in the assay mix in which complex formation is determined.

7. Process according to any of the preceding claims, wherein the solid phase is a particulate solid phase.

8. Process according to Claim 6, wherein formation of a complex between VWF protein, GPIbα protein and the solid phase is determined on the basis of agglutination of the particulate solid phase.

9. Process according to any of Claims 1 to 6, wherein the solid phase is a non-particulate solid phase, preferably the surface of a microtiter plate.

10. Process according to Claim 9, wherein formation of a complex between VWF protein, GPIbα protein and the solid phase is determined on the basis of determining VWF protein bound to the solid phase.

## Revendications

1. Procédé de criblage d'un échantillon de patient ayant une interaction facteur de Von Willebrand-GPIb perturbée, dans lequel
a. on met un échantillon de sang, de plasma ou de sérum d'un patient en contact avec une protéine de GPIbα isolée, avec une protéine du facteur de Von Willebrand et avec une phase solide, qui est associée à un anticorps ayant de la spécificité pour la protéine de GPIbα isolée et
b. on détermine la formation d'un complexe entre la protéine du facteur de Von Willebrand, la protéine de GPIbα et la phase solide.

2. Procédé suivant la revendication 1, dans lequel la protéine de GPIbα est une protéine de GPIbα humaine de type sauvage ou l'un de ses fragments fonctionnels et dans lequel on met l'échantillon en outre en contact avec de la ristocétine ou de la botrocétine.

3. Procédé suivant la revendication 1, dans lequel la protéine de GPIbα est mutée et, par rapport à la séquence de type sauvage de la protéine de GPIbα humaine, contient au moins les restes d'acides aminés 1 à 268 et a, sur au moins deux des positions 233, 235 et 239, respectivement une substitution Xaa (identification de séquence n° 1) et dans lequel on ne met pas l'échantillon en contact avec de la ristocétine ou de la botrocétine.

4. Procédé suivant la revendication 3, dans lequel la protéine de GPIbα mutée a une substitution choisie dans le groupe G233V, G233S, D235Y, M239V et M239S.

5. Procédé suivant l'une des revendications précédentes, dans lequel on met l'échantillon en contact avec une quantité de protéine de GPIbα de manière à obtenir dans la composition de test, dans laquelle on détermine la formation d'un complexe, une concentration finale de moins de 1,4 µg/mL de protéine de GPIbα, d'une manière particulièrement préférée, de moins de 0,7 µg/mL de protéine de GPIbα.

6. Procédé suivant l'une des revendications précédentes, dans lequel on met l'échantillon en contact avec une quantité de protéine du facteur de Von Willebrand de manière à obtenir dans la composition de test, dans laquelle on détermine la formation d'un complexe, une concentration finale de 0,1 à 20 % de l'activité de norme du facteur de Von Willebrand, d'une manière particulièrement préférée, de 0,5 à 10 % de l'activité de norme du facteur de Von Willebrand.

7. Procédé suivant l'une des revendications précédentes, dans lequel la phase solide est en particules.

8. Procédé suivant la revendication 6, dans lequel on détermine la formation d'un complexe entre la protéine du facteur de Von Willebrand, la protéine de GPIbα et la phase solide à l'aide de l'agglutination de la phase solide en particules.

9. Procédé suivant l'une des revendications 1 à 6, dans lequel la phase solide est une phase solide qui n'est pas en particules, en étant de préférence la surface d'une plaquette de microtitration.

10. Procédé suivant la revendication 9, dans lequel on détermine la formation d'un complexe entre la protéine du facteur de Von Willebrand, la protéine de GPIbα et la phase solide à l'aide de la détermination de la protéine du facteur de Von Willebrand fixée par des phases solides.


### SEQUENCE LISTING

<110> Siemens Healthcare Diagnostics Products GmbH
<120> Screeningverfahren zum Auffinden von Proben mit einer Störung der Funktionalität der GPIb-Von Willebrand Faktor-Interaktion
<130> 2011P27081EP01
<150> EP 12158022.9
   <151> 2012-03-05
<160> 3
<170> PatentIn version 3.3
<210> 1
   <211> 610
   <212> PRT
   <213> Homo sapiens
<400> 1
   His Pro Ile Cys Glu Val Ser Lys Val Ala Ser His Leu Glu Val Asn
   1               5                   10                  15
   Cys Asp Lys Arg Asn Leu Thr Ala Leu Pro Pro Asp Leu Pro Lys Asp
               20                  25                  30
   Thr Thr Ile Leu His Leu Ser Glu Asn Leu Leu Tyr Thr Phe Ser Leu
           35                  40                  45
   Ala Thr Leu Met Pro Tyr Thr Arg Leu Thr Gln Leu Asn Leu Asp Arg
       50                  55                  60
   Cys Glu Leu Thr Lys Leu Gln Val Asp Gly Thr Leu Pro Val Leu Gly
   65                  70                  75                  80
   Thr Leu Asp Leu Ser His Asn Gln Leu Gln Ser Leu Pro Leu Leu Gly
                   85                  90                  95
   Gln Thr Leu Pro Ala Leu Thr Val Leu Asp Val Ser Phe Asn Arg Leu
               100                 105                 110
   Thr Ser Leu Pro Leu Gly Ala Leu Arg Gly Leu Gly Glu Leu Gln Glu
           115                 120                 125
   Leu Tyr Leu Lys Gly Asn Glu Leu Lys Thr Leu Pro Pro Gly Leu Leu
       130                 135                 140
   Thr Pro Thr Pro Lys Leu Glu Lys Leu Ser Leu Ala Asn Asn Asn Leu
   145                 150                 155                 160
   Thr Glu Leu Pro Ala Gly Leu Leu Asn Gly Leu Glu Asn Leu Asp Thr
                   165                 170                 175
   Leu Leu Leu Gln Glu Asn Ser Leu Tyr Thr Ile Pro Lys Gly Phe Phe
               180                 185                 190
   Gly Ser His Leu Leu Pro Phe Ala Phe Leu His Gly Asn Pro Trp Leu
           195                 200                 205
   Cys Asn Cys Glu Ile Leu Tyr Phe Arg Arg Trp Leu Gln Asp Asn Ala
       210                 215                 220
   Glu Asn Val Tyr Val Trp Lys Gln Gly Val Asp Val Lys Ala Met Thr
   225                 230                 235                 240
   Ser Asn Val Ala Ser Val Gln Cys Asp Asn Ser Asp Lys Phe Pro Val
                   245                 250                 255
   Tyr Lys Tyr Pro Gly Lys Gly Cys Pro Thr Leu Gly Asp Glu Gly Asp
               260                 265                 270
   Thr Asp Leu Tyr Asp Tyr Tyr Pro Glu Glu Asp Thr Glu Gly Asp Lys
           275                 280                 285
   Val Arg Ala Thr Arg Thr Val Val Lys Phe Pro Thr Lys Ala His Thr
       290                 295                 300
   Thr Pro Trp Gly Leu Phe Tyr Ser Trp Ser Thr Ala Ser Leu Asp Ser
   305                 310                 315                 320
   Gln Met Pro Ser Ser Leu His Pro Thr Gln Glu Ser Thr Lys Glu Gln
                   325                 330                 335
   Thr Thr Phe Pro Pro Arg Trp Thr Pro Asn Phe Thr Leu His Met Glu
               340                 345                 350
   Ser Ile Thr Phe Ser Lys Thr Pro Lys Ser Thr Thr Glu Pro Thr Pro
           355                 360                 365
   Ser Pro Thr Thr Ser Glu Pro Val Pro Glu Pro Ala Pro Asn Met Thr
       370                 375                 380
   Thr Leu Glu Pro Thr Pro Ser Pro Thr Thr Pro Glu Pro Thr Ser Glu
   385                 390                 395                 400
   Pro Ala Pro Ser Pro Thr Thr Pro Glu Pro Thr Pro Ile Pro Thr Ile
                   405                 410                 415
   Ala Thr Ser Pro Thr Ile Leu Val Ser Ala Thr Ser Leu Ile Thr Pro
   420 425 430
   Lys Ser Thr Phe Leu Thr Thr Thr Lys Pro Val Ser Leu Leu Glu Ser
   435 440 445
   Thr Lys Lys Thr Ile Pro Glu Leu Asp Gln Pro Pro Lys Leu Arg Gly
   450 455 460
   Val Leu Gln Gly His Leu Glu Ser Ser Arg Asn Asp Pro Phe Leu His
   465 470 475 480
   Pro Asp Phe Cys Cys Leu Leu Pro Leu Gly Phe Tyr Val Leu Gly Leu
   485 490 495
   Phe Trp Leu Leu Phe Ala Ser Val Val Leu Ile Leu Leu Leu Ser Trp
   500 505 510
   Val Gly His Val Lys Pro Gln Ala Leu Asp Ser Gly Gln Gly Ala Ala
   515 520 525
   Leu Thr Thr Ala Thr Gln Thr Thr His Leu Glu Leu Gln Arg Gly Arg
   530 535 540
   Gln Val Thr Val Pro Arg Ala Trp Leu Leu Phe Leu Arg Gly Ser Leu
   545 550 555 560
   Pro Thr Phe Arg Ser Ser Leu Phe Leu Trp Val Arg Pro Asn Gly Arg
   565 570 575
   Val Gly Pro Leu Val Ala Gly Arg Arg Pro Ser Ala Leu Ser Gln Gly
   580 585 590
   Arg Gly Gln Asp Leu Leu Ser Thr Val Ser Ile Arg Tyr Ser Gly His
   595 600 605
   Ser Leu
   610
<210> 2
   <211> 16
   <212> PRT
   <213> Homo sapiens
<400> 2
   Met Pro Leu Leu Leu Leu Leu Leu Leu Leu Pro Ser Pro Leu His Pro
   1 5 10 15
<210> 3
   <211> 41
   <212> PRT
   <213> Artificial
<220>
   <223> heterologous signal peptide
<400> 3
   Met Pro Leu Gln Leu Leu Leu Leu Leu Ile Leu Leu Gly Pro Gly Asn
   1               5                   10                  15
   Ser Leu Gln Leu Trp Asp Thr Trp Ala Asp Glu Ala Glu Lys Ala Leu
               20                  25                  30
   Gly Pro Leu Leu Ala Arg Asp Arg Arg
           35                  40